# EUROPEAN PATENT SPECIFICATION

(11) **EP 2 123 242 B1**
(45) Date of publication and mention of the grant of the patent: **08.05.2013**
(21) Application number: 08104003.2
(22) Date of filing: 19.05.2008
(51) Int. Cl.: A61F 13/15

(54) **Absorbent core**
Saugfähiger Kern
Noyau absorbant

(43) Date of publication of application: 25.11.2009
(73) Proprietor: The Procter & Gamble Company, Cincinnati, OH 45202 (US)
(72) Inventor: Carlucci, Giovanni, 66100, Chieti (IT); Gagliardini, Alessandro, 65010, Moscufo (Pescara) (IT); Somma, Emma, 65015, Montesilvano (Pescara) (IT); Tamburro, Maurizio, 66020, Sambuceto (Chieti) (IT); Toro, Evelina, 66100 Chieti (IT)
(74) Representative: Briatore, Andrea

(56) References cited:
- EP-A- 1 149 596
- EP-A- 1 447 067
- WO-A-92/19652
- WO-A-96/17681
- AU-B2- 708 191
- US-A- 5 731 259
- US-A1- 2006 240 229
- US-B1- 6 258 996

## Description

### FIELD OF THE INVENTION

The present invention relates to an absorbent core for absorbent articles, for example sanitary napkins and the like.

### BACKGROUND OF THE INVENTION

Absorbent articles for absorption of body fluids such as menses or blood or vaginal discharges are well known in the art, and comprise for example feminine hygiene articles such as sanitary napkins, panty liners, tampons, interlabial devices, as well as wound dressings, and the like. When considering for example sanitary napkins, these articles typically comprise a liquid-pervious topsheet as wearer-facing layer, a backsheet as garment-facing layer and an absorbent core between topsheet and backsheet. The body fluids are acquired through the topsheet and subsequently stored in the absorbent core. The backsheet typically prevents the absorbed fluids from wetting the wearer's garment.

An absorbent core can typically comprise one or more fibrous absorbent material, which in turn can comprise natural fibres, such as for example cellulose fibres, typically wood pulp fibres, synthetic fibres, or combinations thereof.

Absorbent articles can further comprise, typically in the absorbent core, superabsorbent materials, such as absorbent gelling materials (AGM), usually in finely dispersed form, e.g. typically in particulate form, in order to improve their absorption and retention characteristics. Superabsorbent materials for use in absorbent articles typically comprise water-insoluble, water-swellable, hydrogel-forming crosslinked absorbent polymers which are capable of absorbing large quantities of liquids and of retaining such absorbed liquids under moderate pressure. Absorbent gelling materials can be incorporated in absorbent articles, typically in the core structure, in different ways; for example, absorbent gelling materials in particulate form can be dispersed among the fibres of fibrous layers comprised in the core, or rather localized in a more concentrated arrangement between fibrous layers.

Absorbent cores for absorbent articles having a thin structure can further provide an improved immobilization of absorbent gelling materials, particularly when the article is fully or partially loaded with liquid, and an increased wearing comfort. Such thinner structures provide absorbent articles combining better comfort, discreetness and adaptability, such as for example, thin absorbent structures where the absorbent gelling material is located and somehow kept in selected, e.g. patterned regions of the structure itself.

EP 1447067, assigned to the Procter & Gamble Company, describes an absorbent article, typically a disposable absorbent article, such as a diaper, having an absorbent core which imparts increased wearing comfort to the article and makes it thin and dry. The absorbent core comprises a substrate layer, the substrate layer comprising a first surface and a second surface, the absorbent core further comprising a discontinuous layer of absorbent material, the absorbent material comprising an absorbent polymer material, the absorbent material optionally comprising an absorbent fibrous material which does not represent more than 20 weight percent of the total weight of the absorbent polymer material. The discontinuous layer of absorbent material comprises a first surface and a second surface, the absorbent core further comprising a layer of thermoplastic material, the layer of thermoplastic material comprising a first surface and a second surface and wherein the second surface of the discontinuous layer of absorbent material is in at least partial contact with the first surface of the substrate layer and wherein portions of the second surface of the layer of thermoplastic material are in direct contact with the first surface of the substrate layer and portions of the second surface of the layer of thermoplastic material are in direct contact with the first surface of the discontinuous layer of absorbent material.

While absorbent articles according to EP 1447067 and comprising thin absorbent cores with relatively high amounts of absorbent gelling materials and rather low content of fibrous materials commonly have good absorption and retention characteristics to body fluids like urine, there still remains room for improvement of absorption and retention, particularly towards other body fluids. In particular, menses, blood and vaginal discharges are particularly difficult to be effectively absorbed and retained into absorbent cores containing superabsorbent materials in major amounts since such materials may not show optimal absorption and retention characteristics towards such body fluids. Particularly, superabsorbent materials may show a relatively slow acquisition and absorption rate.

It is believed that the non-optimal absorption and retention are mainly caused by poor permeability of superabsorbent materials towards menses, blood or vaginal discharges due to the viscosity and/or to the complex nature of these fluids. For example menses and blood are water based fluids comprising components having molecular weights higher than water and also corpuscular components, including red cells, white cells, soluble proteins, cellular debris and mucus, which slow down the absorption of these fluids by superabsorbents. Menses and blood are rather thick, and more difficult to absorb in conventional absorbent structures comprising absorbent gelling materials; moreover, corpuscular components like red cells may decrease the absorption capacity of certain superabsorbent particles. This translates into a slower initial uptake rate of the fluid into the superabsorbent material, and in turn in the absorbent structure comprising the superabsorbent material, which can result in a lower final absorption and retention capacity.

Cationic polysaccharides are mentioned in absorbent articles, for example typically in order to provide ion exchange or odour control, such as in US 6 258 996, AU 708 191, US 5731259, EP 1149596, US 2006/0240229.

The present invention provides significant improvements in the above area by the incorporation of cationic polysaccharides in an absorbent core structure for an absorbent article, particularly for absorption of menses or blood or vaginal discharges, which comprises the absorbent gelling material in a non uniform layer stably provided onto a fibrous substrate layer.

### SUMMARY OF THE INVENTION

The present invention addresses the above needs by providing an absorbent core for an absorbent article intended for absorption of menses or blood or vaginal discharges, as defined in the ensuing claims.

### BRIEF DESCRIPTION OF THE DRAWINGS

Figure 1 is a plan view of a sanitary napkin showing an absorbent core according to an embodiment of the present invention, with portions of some constituent elements cut out in order to show underlying elements.
Figure 2 is a schematic cross section of the sanitary napkin of Figure 1 taken in the transverse axis A-A'.
Figure 3 shows a schematic cross section of an absorbent core according to one embodiment of the present invention.
Figure 4 shows a schematic cross section of an absorbent core according to another embodiment of the present invention.
Figure 5 shows a perspective view of an exemplary absorbent core according to the present invention.

### DETAILED DESCRIPTION OF THE INVENTION

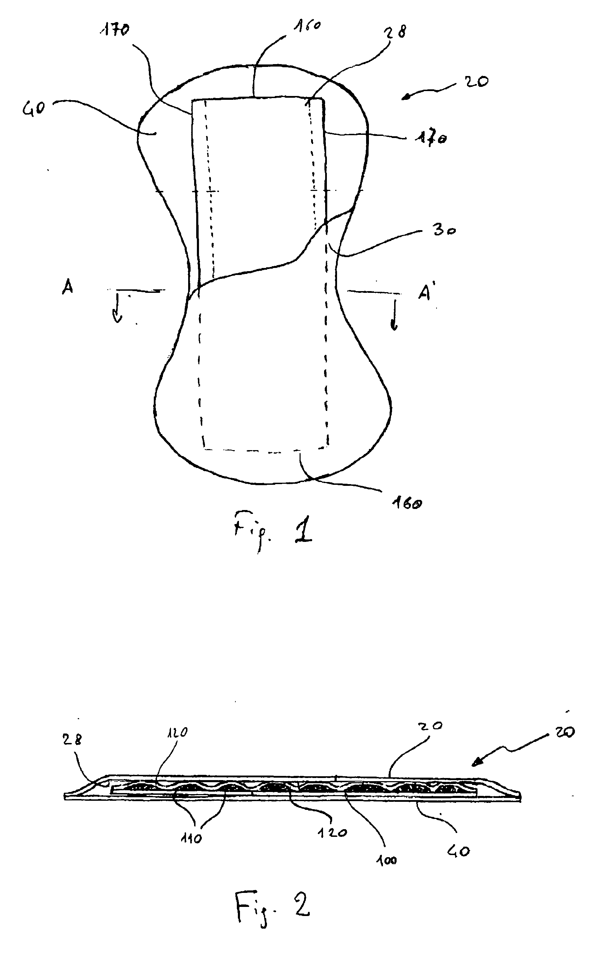

The present invention relates to an absorbent core for absorbent articles such as sanitary napkins, panty liners, tampons, interlabial devices, wound dressings, and the like, which are intended for the absorption of body fluids, such as menses or blood or vaginal discharges. Exemplary absorbent articles in the context of the present invention are disposable absorbent articles. The term "disposable" is used herein to describe articles, which are not intended to be laundered or otherwise restored or reused as an article (i.e. they are intended to be discarded after a single use and preferably to be recycled, composted or otherwise disposed of in an environmentally compatible manner). The absorbent core of the present invention will be herein described in the context of a typical absorbent article, such as, for example, a sanitary napkin 20 as illustrated in Figure 1. Typically, such articles as shown in Figure 1 can comprise the elements of a liquid pervious topsheet 30, a backsheet 40 and an absorbent core 28 intermediate said topsheet 30 and said backsheet 40.

In the following description of the invention, the surface of the article, or of each element thereof, which in use faces in the direction of the wearer is called wearer-facing surface. Conversely, the surface facing in use in the direction of the garment is called garment-facing surface. The absorbent article of the present invention, as well as any element thereof, such as, for example the absorbent core, has therefore a wearer-facing surface and a garment-facing surface.

### Topsheet

According to the present invention, the absorbent article can comprise a liquid pervious topsheet. The topsheet suitable for use herein can comprise wovens, non-wovens, and/or three-dimensional webs of a liquid impermeable polymeric film comprising liquid permeable apertures. In Figure 1 the topsheet is indicated with reference numeral 30. The topsheet for use herein can be a single layer or may have a multiplicity of layers. For example, the wearer-facing and contacting surface can be provided by a film material having apertures which are provided to facilitate liquid transport from the wearer facing surface towards the absorbent structure. Such liquid permeable, apertured films are well known in the art. They provide a resilient three-dimensional fibre-like structure. Such films have been disclosed in detail for example in US 3929135, US 4151240, US 4319868, US 4324426, US 4343314, US 4591523, US 4609518, US 4629643, US 4695422 or WO 96/00548.

### Absorbent Core

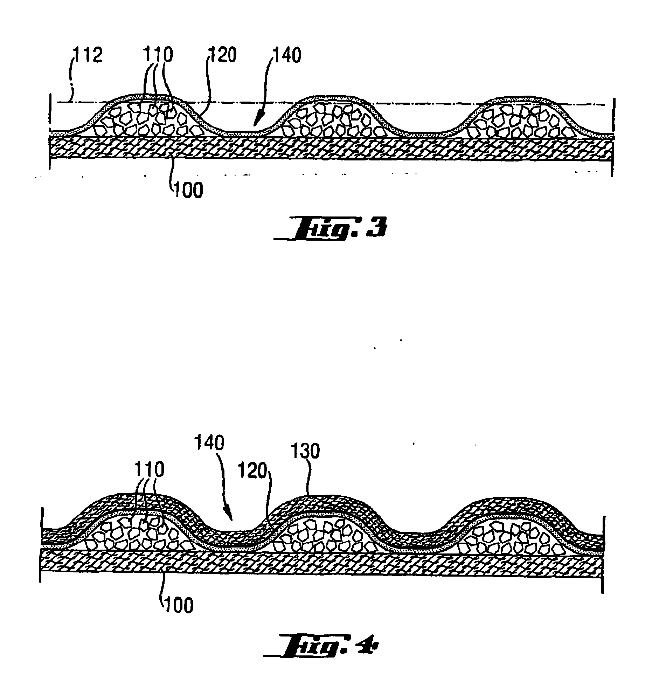

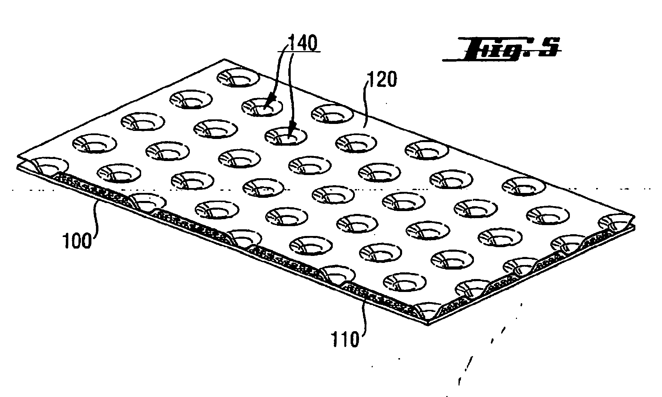

According to the present invention, and as shown for example in the embodiments of Figures 3 and 5, the absorbent core 28 can comprise a substrate layer 100, absorbent polymer material 110, as layer of thermoplastic material 120, a layer of fiberized hot melt adhesive 120. The substrate layer 100 is provided from a fibrous material, as will be explained in detail below.

An alternative embodiment of the present invention is shown in Figure 4. The absorbent core shown in Figure 4 can further comprise a cover layer 130. This cover layer may be provided of the same material as the substrate layer 100, or may be provided from a different material. Suitable materials for the cover layer are for example nonwoven materials, as will be better explained further on.

The substrate layer 100 comprises a first surface and a second surface. Conventionally, in the sectional views illustrated in the attached drawings the first surface of each layer can be said to correspond to the top surface, in turn the wearer facing surface of the article 20, while the second surface corresponds to the bottom surface, in turn the garment facing surface. At least portions of the first surface of the substrate layer 100 are in contact with a layer of absorbent polymer material 110. This layer of absorbent polymer material 110 can be typically a non uniform layer, and comprises a first surface and a second surface, wherein by "non uniform" it is meant that the absorbent polymer material 110 is distributed over the substrate layer 100 with non uniform basis weight. Conversely, the second surface of the non uniform layer of absorbent polymer material 110 is in at least partial contact with the first surface of the substrate layer 100. According to an embodiment of the present invention, the non uniform layer of absorbent polymer material 110 can be a discontinuous layer that is a layer typically comprising openings, i.e. areas substantially free of absorbent polymer material, which in certain embodiments can be typically completely surrounded by areas comprising absorbent polymer material, as will be explained in more detail later on. Typically these openings have a diameter or largest span of less than 10 mm, or less than 5 mm, or 3 mm, or 2 mm, or 1.5 mm and of more than 0.5 mm, or 1 mm. At least portions of the second surface of the absorbent polymer material layer 110 are in contact with at least portions of the first surface of the substrate layer material 100. The first surface of the absorbent polymer material 110 defines a certain height of the layer of absorbent polymer material above the first surface of the layer of substrate material 100. When the absorbent polymer material layer 110 is provided as a non uniform layer, typically for example as a discontinuous layer, at least some portions of the first surface of the substrate layer 100 are not covered by absorbent polymer material 110. The absorbent core 28 further comprises a layer of a thermoplastic material 120. This thermoplastic material 120 serves to at least partially immobilize the absorbent polymer material 110.

The thermoplastic material 120 is provided as a fibrous layer which is partially in contact with the absorbent polymer material 110 and partially in contact with the substrate layer 100. Figures 3 and 5 show such a structure in an exemplary embodiment of the present invention. In this structure the absorbent polymer material layer 110 is provided as a discontinuous layer, a layer of fiberized thermoplastic material 120 is laid down onto the layer of absorbent polymeric material 110, such that the thermoplastic layer 120 is in direct contact with the first surface of the layer of absorbent polymer material 110, but also in direct contact with the first surface of the substrate layer 100, where the substrate layer is not covered by the absorbent polymeric material 110, i.e. typically in correspondence of the openings of the discontinuous layer of the polymer material 120. By "direct contact" it is meant that there is no further intermediate component layer between the layer of thermoplastic material 120 and the other respective layer in direct contact thereto, such as for example a further fibrous layer. It is however not excluded that a further adhesive material can be comprised between the layer of thermoplastic material 120 and the optional cover layer 130, when present, as shown in Figure 4, or the layer of absorbent polymer material 110 or, more typically, the substrate layer 100, such as for example a supplementary adhesive material provided onto the first surface of the substrate layer 100 to further stabilize the overlying absorbent polymer material 110. "Direct contact" can hence be considered to mean in this context a direct adhesive contact between the layer of thermoplastic material 120 and the other respective layer as explained above. This imparts an essentially three-dimensional structure to the fibrous layer of thermoplastic material 120 which in itself is essentially a two-dimensional structure of relatively small thickness (in z-direction), as compared to the extension in x- and y-direction. In other words, the fibrous thermoplastic material layer 120 undulates between the first surface of the absorbent polymer material 110 and the first surface of the substrate layer 100. The areas where the fibrous thermoplastic material 120 is in contact with the substrate layer 100 are the areas of junction 140.

Thereby, the thermoplastic material 120 provides spaces to hold the absorbent polymer material 110 typically towards the substrate layer 100, and thereby immobilizes this material. In a further aspect, the thermoplastic material 120 bonds to the substrate 100 and thus affixes the absorbent polymer material 110 to the substrate 100. Typical thermoplastic materials will also penetrate into both the absorbent polymer material 110 and the substrate layer 100, thus providing for further immobilization and affixation.

In the alternative embodiment representatively illustrated in Figure 4 portions of the cover layer 130 bond to portions of the substrate layer 100 via the thermoplastic material 120. Thereby, the substrate layer 100 together with the cover layer 130 provides spaces to immobilize the absorbent polymer material 110.

Of course, while the thermoplastic materials disclosed herein can provide a much improved wet immobilisation, i.e. immobilisation of absorbent polymer material when the article is wet or at least partially loaded, these thermoplastic materials can also provide a very good immobilisation of absorbent polymer material when the article is dry.

In accordance with an embodiment of the present invention, the absorbent polymer material 110 may also be optionally mixed with fibrous material, which can provide a matrix for further immobilization of the absorbent polymer material. However, typically a relatively low amount of fibrous material can be used, for example less than 40 weight %, less than 20 weight %, or less than 10 weight % of the total weight of the absorbent polymer material 110, positioned within the areas of absorbent polymer material.

According to an embodiment of the present invention, in a typically discontinuous layer of absorbent polymer material 110 the areas of absorbent polymer material can be connected to one another, while the areas of junction 140 can be areas, which in an embodiment may correspond to the openings in the discontinuous layer of absorbent polymer material, as shown for example in Figure 5. The areas of absorbent polymer material are then referred to as connected areas. In an alternative embodiment, the areas of junction 140 can be connected to one another. Then, the absorbent polymer material can be deposited in a discrete pattern, or in other words the absorbent polymer material represents islands in a sea of thermoplastic material 120. Hence, in summary, a discontinuous layer of absorbent polymer material 110 may comprise connected areas of absorbent polymer material 110, as e.g. illustrated in Figure 5, or may alternatively comprise discrete areas of absorbent polymer material 110.

The present invention, and specifically the embodiments described with reference to Figures 3, 4 and 5 can be used to provide a storage layer of an absorbent core. However, they can also be used to provide the full absorbent core 28 as illustrated in Figure 1. In that case, no further materials wrapping the core, such as for example a top layer and a bottom layer are being used. With reference to the embodiments of Figure 4 the optional cover layer 130 may provide the function of a top layer and the substrate layer 100 may provide the function of a bottom layer of an absorbent core, wherein top and bottom layers respectively correspond to the body facing and garment facing surfaces of the core 28.

With reference to Figures 3, 4 and 5 the areas of direct contact between the thermoplastic material 120 and the substrate material 100 are referred to as areas of junction 140. The shape, number and disposition of the areas of junction 140 will influence the immobilization of the absorbent polymer material 110. The areas of junction can be for example of squared, rectangular or circular shape. Areas of junction of circular shape can have a diameter of more than 0.5 mm, or more than 1 mm, and of less than 10 mm, or less than 5 mm, or less than 3 mm, or less than 2 mm, or less than 1.5 mm. If the areas of junction 140 are not of circular shape, they can be of a size as to fit inside a circle of any of the diameters given above.

The areas of junction 140 can be disposed in a regular or irregular pattern. For example, the areas of junction 140 may be disposed along lines as shown in Figure 5. These lines may be aligned with the longitudinal axis of the absorbent core, or alternatively they may have a certain angle in respect to the longitudinal edges of the core. A disposition along lines parallel with the longitudinal edges of the absorbent core 28 might create channels in the longitudinal direction which can lead to a lesser wet immobilization, hence for example the areas of junction 140 can be arranged along lines which form an angle of 20 degrees, or 30 degrees, or 40 degrees, or 45 degrees with the longitudinal edges of the absorbent core 28. Another pattern for the areas of junction 140 can be a pattern comprising polygons, for example pentagons and hexagons or a combination of pentagons and hexagons. Also typical can be irregular patterns of areas of junction 140, which also can give a good wet immobilization. Irregular patterns of areas of junction 140 can also give a better fluid handling behaviour in case of absorption of menses or blood or vaginal discharges, since fluid can start diffusing in whichever direction from any initial acquisition point with substantially the same probability of contacting the absorbent polymer material in the e.g. discontinuous layer. Conversely, regular patterns might create preferential paths the fluid could follow with lesser probability of actually contacting the absorbent polymer material.

According to the present invention the thermoplastic layer is a hot melt adhesive in fiberized form. A variety of thermoplastic materials can be suitable to immobilize the absorbent polymer material. Some initially thermoplastic materials may later lose their thermoplasticity due to a curing step, e.g. initiated via heat, UV radiation, electron beam exposure or moisture or other means of curing, leading to the irreversible formation of a crosslinked network of covalent bonds. Those materials having lost their initial thermoplastic behaviour can be herein also understood as thermoplastic materials 120.

Without wishing to be bound by theory it has been found that those thermoplastic materials, i.e. the hot melt adhesives, can be most useful for immobilizing the absorbent polymer material 110, which combine good cohesion and good adhesion behaviour. Good adhesion is critical to ensure that the thermoplastic layer 120 maintains good contact with the absorbent polymer material 110 and in particular with the substrate material 100. Good adhesion is a challenge, namely when a non-woven substrate material is used. Good cohesion ensures that the adhesive does not break, in particular in response to external forces, and namely in response to strain. The adhesive is subject to external forces when the absorbent product has acquired liquid, which is then stored in the absorbent polymer material 110 which in response swells. An exemplary adhesive should allow for such swelling, without breaking and without imparting too many compressive forces, which would restrain the absorbent polymer material 110 from swelling. It may be desirable that the adhesive not break, which would deteriorate the wet immobilization. Exemplary suitable thermoplastic materials can be as described in the already mentioned patent application EP 1447067, particularly at sections [0050] to [0063].

The hotmelt adhesive, is present in the form of fibres throughout the core, being provided with known means, i.e. the adhesive is fiberized. Typically, the fibres can have an average thickness of 1 - 100 micrometer and an average length of 5 mm to 50 cm. In particular the layer of hot melt adhesive, can be provided such as to comprise a net-like structure.

To improve the adhesiveness of the thermoplastic material 120 to the substrate layer 100 or to any other layer, in particular any other non-woven layer, such layers may be pretreated with an auxiliary adhesive.

In particular, typical parameters of a hot melt adhesive in accordance with the present invention can be as follows.

In an aspect, the loss angle tan Delta of the adhesive at 60°C should be below the value of 1, or below the value of 0.5. The loss angle tan Delta at 60°C is correlated with the liquid character of an adhesive at elevated ambient temperatures. The lower tan Delta, the more an adhesive behaves like a solid rather than a liquid, i.e. the lower its tendency to flow or to migrate and the lower the tendency of an adhesive superstructure as described herein to deteriorate or even to collapse over time. This value is hence particularly important if the absorbent article is used in a hot climate.

In a further aspect, hot melt adhesives in accordance with the present invention may have a sufficient cohesive strength parameter γ. The cohesive strength parameter γ is measured using the Rheological Creep Test as referred to hereinafter. A sufficiently low cohesive strength parameter γ is representative of elastic adhesive which, for example, can be stretched without tearing. If a stress of τ = 1000 Pa is applied, the cohesive strength parameter γ can be less than 100%, less than 90%, or less than 75%. For a stress of τ = 125000 Pa, the cohesive strength parameter γ can be less than 1200%, less than 1000%, or less than 800%.

In the absorbent core of the present invention the substrate layer 100 and the optional cover layer 130 can be typically provided from nonwoven materials, for example spunbonded or carded nonwoven materials, or also airlaid materials, such as for example latex and/or thermal bonded airlaid materials.

Exemplary materials for the substrate layer 100 can comprise fibrous materials comprising cellulose fibres, typically not more than 60% by weight of cellulose fibres, or from 30% to 50% by weight of cellulose fibres. Examples of fibrous materials for the substrate layer 100 can be nonwoven materials, such as for example carded nonwovens, or more typically airlaid or wetlaid fibrous materials, such as for example latex or thermal bonded airlaid fibrous materials, comprising synthetic and natural fibres, such as for example cellulose fibres. Basis weights for the materials of the substrate layer 100 can typically range from 10 g/m² to 120 g/m², or from 40 g/m² to 100 g/m², or also from 50 g/m² to 80 g/m².

Exemplary materials for the optional cover layer 130 can be provided by nonwoven materials comprising synthetic fibres, such as polyethylene (PE), polyethylene terephthalate (PET), polypropylene (PP). As the polymers used for nonwoven production are inherently hydrophobic, they can be typically coated with hydrophilic coatings, for example with durably hydrophilic coatings to provide permanently hydrophilic nonwovens. Other nonwoven materials for the optional cover layer 130 can comprise composite structures such as a so called SMS material, comprising a spunbonded, a melt-blown and a further spunbonded layer. Basis weights for the materials of the cover layer 130 can typically range from 5 g/m² to 80 g/m², or from 10 g/m² to 60 g/m², or also from 20 g/m² to 40 g/m²

In certain embodiments of the present invention the absorbent polymer material 110 in the absorbent core 28 is present throughout the area of the absorbent core in an average basis weight of less than 220 g/m², of less than 180 g/m², from 60 g/m² to 150 g/m², or from 80 g/m² to 120 g/m². An average basis weight is typically based on the whole area of the zone of application, i.e. interested by the layer of absorbent polymer material, and hence comprising possible openings included in an e.g. discontinuous layer.

According to the present invention, the absorbent core further comprises a cationic polysaccharide 150, or a mixture thereof comprised in a carrier material.

According to the present invention the absorbent core comprises a cationic polysaccharide or a mixture of cationic polysaccharides.

Suitable cationic polysaccharides for use herein are positively charged polysaccharides due to the presence of cationic functional groups. Suitable polysaccharides for use herein include natural and semi-synthetic cationic polysaccharides. Suitable for use herein are any aminopolysaccharide-based polymer with cationic amino functional groups or any quaternary ammonium polysaccharide-based polymer with cationic quaternary functional groups. Examples of suitable cationic functional groups include primary, secondary or tertiary amine groups or quaternary ammonium groups. Preferably quaternary ammonium groups are present.

The cationic polysaccharides for use herein might be a fibrous polysaccharide such as cellulose with an excess of quaternary ammonium compound containing at least one group capable of reacting with polysaccharide hydroxyl groups. Such cationic polysaccharides are described in WO 92/19652 and WO 96/17681.

Suitable cationic polysaccharides for use herein can typically have a pH in the range 4 to 9, or from 5 to 7.5. By pH of cationic polysaccharides it is meant herein the pH of a 1% polysaccharide solution (1 gram of polysaccharide material dissolved in 100 grams of distilled water) measured by pH-meter.

Typical polysaccharides for use in the present invention can be aminopolysaccharides, namely chitin-based materials, chitosan materials, aminocellulose and mixtures thereof. By "chitosan material" it is meant herein chitosan, modified chitosans, crosslinked chitosan and chitosan salts. Suitable chitosan materials for use in the present invention can be those described for example in patents EP-B-1276512, paragraphs [0029] to [0062], and EP-B-1300164, paragraphs [0031] to [0051], assigned to The Procter & Gamble Company.

Suitable chitosan materials are commercially available from numerous vendors. Exemplary chitosan materials are those available for example from Halo Source, Inc. WA, USA, formerly Vanson Halo Source, Inc.

Other typical cationic polysaccharides for use in the present invention can be cationic starches typically comprising quaternary ammonium groups, or amino groups, or mixtures thereof. Various methods for making cationic modified starches are known, see for example US 2,813,093 and US 4,281,109.

The term "cationic modified starch" as used herein refers to the product of the reaction between starch and a suitable cationizing agent.

The source of starch before chemical modification can be chosen from any usual sources including tubers, legumes, cereal, and grains. Non-limiting examples of this source starch may include corn starch, wheat starch, rice starch, waxy corn starch, oat starch, cassaya starch, waxy barley, tapioca starch, potato starch or mixtures thereof.

Starch, particularly native starch, comprises polymers made of glucose units. There are two distinct polymer types. One type of polymer is amylose whereas the other is amylopectin. In one embodiment, the cationic starch of the present invention can comprise a starch comprising amylopectin at a level of from about 90-100% Wt and more specifically above 95% Wt.

Suitable cationizing agents comprising an ammonium group may include those listed in US 5,780,616 from column 4 line 5 to column 5 line 15, and typically substituents such as:
- 2,3-epoxypropyltrimethyl ammonium chloride (CAS # 3033-77-0), having for structural formula: 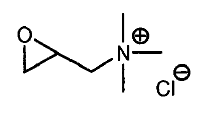
- N-(3-chloro-2-hydroxypropyl) trimethyl ammonium chloride (CAS # 3327-22-8, for example available from Degussa as Quab 188), having the structural formula: 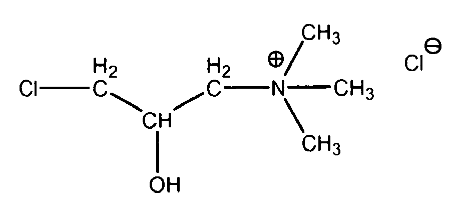
- diethylaminoethylchloride hydrochloride ("DEC", CAS # 869-24-9), having the structural formula:

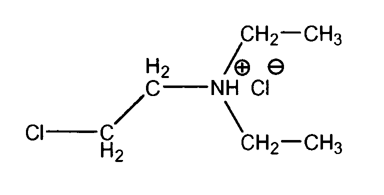

In the absorbent core of the present invention the non uniform layer of absorbent polymer material 110 can at most typically comprise a relatively low amount of fibrous material, or possibly none at all, as explained above, hence all or nearly all absorbent capacity in the absorbent core of the present invention is typically provided by the absorbent polymer material 110 comprised in the non uniform layer. While the absorbent polymer material can typically have a high absorption capacity, it can show a rather slow acquisition capacity and absorption rate, particularly towards complex body fluids such as menses or blood or vaginal discharges. The cationic polysaccharide 150, or mixture of cationic polysaccharides, comprised in the absorbent core of the present invention, can immobilize the body fluid upon contacting it, typically by gelling it or by increasing its viscosity. The reaction time in this process can be rather fast and, without being bound by any theory, it is believed the cationic polysaccharide, or mixture of cationic polysaccharides, can be capable of providing an effective immobilization of the body fluid at a rate which is typically faster than the absorption rate of the absorbent polymer material 110. Hence in use, in the absorbent core of the present invention the body fluid can be effectively handled by the combined action of the absorbent polymer material 110 and of the cationic polysaccharide 150, or mixture of cationic polysaccharides, wherein it can be promptly immobilized by the cationic polysaccharide 150, upon contact therewith, while being acquired and absorbed by the absorbent polymer material 110 over a relatively longer time. This can typically reduce or eliminate the risk of fluid leakage or rewetting, which could in principle be caused by fluid still "free" within the structure of an absorbent core similar to that of the present invention, i.e. typically thin and usually free, or with only a relatively minor amount, of fibrous material specifically meant for fluid absorption, and without the cationic polysaccharide material, during the relatively slow absorption of the fluid by the absorbent polymer material 110.

Further typical cationic polysaccharides for use in the present invention can also be selected among cationic guar gums, typically comprising quaternary ammonium groups, or amino groups, or mixtures thereof.

The cationic polysaccharide material 150, or mixture of cationic polysaccharide materials, can be comprised in the absorbent core of the present invention in different suitable forms, typically depending on how it is actually provided, for example as a powder, or in form of fibres, or particles. The cationic polysaccharide material 150 can be for example comprised within the non uniform layer of absorbent polymer material 110. Alternatively, the polysaccharide material 150 can be comprised in the substrate layer 100, or in the optional cover layer 130, if present, or in both. The polysaccharide material 150 is provided in the absorbent core of the present invention with non uniform distribution, as will be explained more in detail further on.

The absorbent core of the present invention can typically have an oblong shape, for example rectangular, as shown in Figure 1, with a minor dimension and a major dimension. When typically comprised in an absorbent article such as the sanitary napkin 20 of Figure 1, the major dimension and the minor dimension are usually respectively parallel to the longitudinal and the transverse axes of the absorbent article, as clearly visible in the embodiment of Figure 1; the outer perimeter of the absorbent core can hence typically comprise front and rear transverse ends 160, running substantially parallel to the minor dimension thereof, and corresponding within the absorbent article to respective front and rear portions, and longitudinal side ends 170, running substantially parallel to the major dimension thereof, and corresponding in the absorbent article 20 to the side margins. The polysaccharide material 150 is be provided in the absorbent core 28 in areas running substantially along the longitudinal side ends 170 and indicated with dotted lines in Figure 1, over a width of 1 mm to 20 mm, or of 5 mm to 15 mm; the width of the respective areas can be substantially constant along the whole length of the longitudinal side ends 170, or can vary.

The cationic polysaccharide 150, or mixture of cationic polysaccharides, can be provided to the absorbent core in different forms; for example, in case it is water soluble, it can be provided in the desired amount and position as an aqueous solution, sprayed for example onto the fibrous substrate layer 100; upon water evaporation the cationic polysaccharide remains in the fibrous material of the respective layer in the selected amount.

The cationic polysaccharide 150, or mixture of cationic polysaccharides, is provided within a suitable carrier material, for example homogeneously distributed therein. The carrier material can be selected according to patent application EP 1749508, assigned to The Procter & Gamble Company, and can be for example an inert hydrophilic organic carrier which is typically solid at room temperature, wherein by "inert", as referred to the hydrophilic organic carrier, it is meant a hydrophilic organic carrier material which is substantially non reactive with the cationic polysaccharide dispersed therein. The carrier material can be for example selected among polyethylene glycols, polypropylene glycols, and derivatives thereof, such as for example polyoxymethylene glycols. A typical carrier material can be for example a polyethylene glycol having a molecular weight of at least 1200, or between 1200 and 8000, or between 1500 and 4000, or also between 1500 and 2000. Alternatively, a typical carrier material can be a mixture of polyethylene glycols. Comprising the cationic polysaccharide within a carrier material can have the advantage of allowing a simpler and possibly more precise way of providing it in an absorbent core in the selected amount and position.

In certain embodiments of the absorbent core 28 of the present invention the cationic polysaccharide 150, or the mixture of cationic polysaccharides, can be present in the absorbent core in an average basis weight of from 0.5 g/m² to 500 g/m², or from 1 to 200 g/m², or from 3 g/m² to 100 g/m², or also from 4 g/m² to 50 g/m², by weight of the cationic polysaccharide per square meter of the zone of application. An average basis weight is therefore typically based on the area actually interested by the application of the cationic polysaccharide, hence for example in the areas running along the longitudinal side edges of the absorbent core, as explained above with reference to an embodiment of the present invention.

According to the present invention, the absorbent core can provide a more efficient fluid management, in terms of acquisition, immobilization and absorption, as explained above, which can be particularly useful in case of complex body fluids such as menses or blood. Overall, this increased efficiency in the composite structure according to the present invention can translate in a more effective exploitation of the absorbent capacity of the absorbent polymer material, also in presence of problematic body fluids such as menses or blood or vaginal discharges.

This is achieved in a structure which is typically thin and is capable of employing more completely the absorption and immobilization capacity of the different materials, particularly the absorbent polymer material which can hence be present in a typically lesser amount, in synergy with the cationic polysaccharide or polysaccharides, thus overall also providing a particularly thin structure having improved dimensional stability during absorption and therefore increased comfort during use.

According to an embodiment of the present invention the absorbent polymer material can be selected among the polyacrylate based polymers described in the PCT Patent Application WO2007/047598, which are polyacrylate based materials very slightly crosslinked, or substantially not crosslinked at all, this further improving the above mentioned synergistic effect. Particularly, said polyacrylate based materials can have an extractable fraction of at least about 30% by weight, between 30% and 80% by weight, or between 32% and 70% by weight, evaluated according to the Extractables test method described in the above referenced application. Alternatively, said polyacrylate based materials can have a retention capacity of at least about 30 g/g, at least about 35 g/g, or at least about 40 g/g, evaluated according to the Centrifuge Retention Capacity test described in the above referenced application. Said polymers in fact are particularly effective in absorbing complex body fluids such as menses or blood, and upon absorption of such fluids do not generally show a marked swelling, followed by gel blocking, like traditional superabsorbents, but rather act to a certain extent as thickeners of the body fluid, immobilizing it as a sort of gelatinous mass within the absorbent structure, for example in the interstices among the fibres, without causing substantial swelling and in turn a sensible increase of the overall thickness of the absorbent core.

According to the present invention, the absorbent core 28 can fully constitute the absorbent element in an absorbent article, or can constitute part of it, being complemented with other layers in a composite structure. Also, an absorbent article comprising an absorbent core according to the present invention can further comprise a fibrous acquisition layer between the absorbent core 28 and the topsheet. According to an embodiment of the present invention the acquisition layer can for example comprise fibrous nonwoven materials made by air laying or wet laying of synthetic fibres such as polyethylene (PE), polyethylene terephthalate (PET), or polypropylene (PP), similarly to the cover layer 130 of the absorbent core 28 of the present invention.

Exemplary materials for the fluid acquisition layer could comprise spunbonded or carded nonwoven materials, or airlaid materials such as for example latex bonded or thermal bonded airlaid materials. Basis weights can typically range from 10 g/m² to 60 g/m², or from 25 g/m² to 40 g/m².

According to another alternative embodiment of the present invention the absorbent article can comprise a further fibrous layer comprised between the absorbent core 28 and the backsheet, i.e. typically provided at the garment facing surface of the core. This optional layer can be provided by similar fibrous materials as those already described for the substrate layer 100 of the absorbent core of the present invention. This optional fibrous layer according to this further embodiment of the present invention can act as an added wicking layer receiving and distributing excess fluid which might not be fully retained by the absorbent core 28. The presence of cellulose fibres can make the layer particularly effective in acquiring and diffusing the fraction of body fluids like menses or blood which is not completely absorbed by the absorbent polymer material of the absorbent core 28.

An exemplary process for producing absorbent cores 28 in accordance with the present invention can comprise the following steps.

In one step, the substrate layer 100 is laid onto a formation surface. The absorbent polymeric material 110 is disposed by means known in the art, for example by means of a lay-down drum, in the selected non uniform e.g. discontinuous layer onto the substrate layer 100, optionally after providing a stabilizing adhesive on the substrate layer 100, for example in longitudinal stripes. In a further process step, a hot melt adhesive is placed with known means onto the absorbent polymer material, for example in form of fibres.

While any adhesive application means known in the art can be used to place the hot melt adhesive onto the absorbent polymer material, the hot melt adhesive can be typically applied by a nozzle system. For example, a nozzle system can be utilised, which can provide a relatively thin but wide curtain of adhesive, for example in form of fibres. This curtain of adhesive is than placed onto the substrate layer 100 and the absorbent polymer material 110.

In a further process step, an optional cover layer 130 can be typically placed upon the substrate layer 100, the absorbent polymer material and the hot melt adhesive layer. The cover layer 130 will be in adhesive contact with the substrate layer 100 in the areas of junction 140. In these areas of junction 140 the adhesive is in direct contact with the substrate layer 100. The cover layer 130 will typically not be in direct adhesive contact with the substrate layer 100 where the absorbent polymer material 110 is present.

In one alternative embodiment, the cover layer 130 and the substrate layer 100 can be provided from a unitary sheet of material. The placing of the cover layer 130 onto the substrate layer 100 can then involve the folding of the unitary piece of material.

Hence, the uneven service of the lay-down system, which may be a lay-down drum, typically determines the distribution of absorbent polymer material in the non uniform, for example discontinuous layer and likewise can determine the pattern of areas of junction 140. The distribution of absorbent polymer material may be influenced by vacuum means.

The cationic polysaccharide 150, or the mixture of cationic polysaccharides, can be provided in a process step for example as a powder, or particles, or fibres, directly to the non uniform layer of absorbent gelling material, for example intermixed with it in the same lay-down system. Alternatively, the cationic polysaccharide material 150 can be provided as an aqueous solution, or also in a suitable carrier as explained above, for example by spraying the aqueous solution or by applying the carrier material typically in the molten state comprising the cationic polysaccharide onto the substrate layer 100, or onto the cover layer 130, when present, or on both, in selected areas.

The distribution of absorbent polymer material can be profiled, for example profiled in the longitudinal direction, or in the lateral direction, or in both, for example being substantially absent in an area along the longitudinal side ends of the absorbent core, as explained above.

Typically the absorbent polymer material for the absorbent cores according to the present invention can comprise absorbent polymer particles. Without whishing to be bound by theory it is believed that such material, even in the swollen state, i.e. when liquid has been absorbed, does not substantially obstruct the liquid flow throughout the material, particularly when further the permeability of said material, as expressed by the saline flow conductivity of the absorbent polymer material, is greater than 10, 20, 30 or 40 SFC-units, where 1 SFC unit is 1 x 10⁻⁷ (cm³ x s) / g. Saline flow conductivity is a parameter well recognised in the art and is to be measured in accordance with the test disclosed in EP 752 892 B.

### Backsheet

The absorbent article comprising the core according to the present invention can also comprise a backsheet 40. The backsheet primarily has to prevent the fluids absorbed and contained in the absorbent structure from wetting materials that contact the absorbent article such as underpants, pants, pyjamas, undergarments, and shirts or jackets, thereby acting as a barrier to fluid transport. The backsheet according to an embodiment of the present invention can also allow the transfer of at least water vapour, or both water vapour and air through it.

Especially when the absorbent article finds utility as a sanitary napkin or panty liner, the absorbent article can be also provided with a panty fastening means, which provides means to attach the article to an undergarment, for example a panty fastening adhesive on the garment facing surface of the backsheet. Wings or side flaps meant to fold around the crotch edge of an undergarment can be also provided on the side edges of the napkin.

### Example

A sanitary napkin comprising an absorbent core according to an embodiment of the present invention is similar to that illustrated in Figures 1 and 2 and comprises a topsheet constituted by a polyethylene perforated formed film, a backsheet constituted by a 25 g/m² polyethylene film, a core comprising a cover layer constituted by a 30 g/m² carded nonwoven comprising polyester fibres and PP/PE bicomponent fibres, available from BBA Fiberweb under the code TBPL 50/50 6dpf philic PET/BICO, a discontinuous layer of absorbent polymer material constituted by a particulate superabsorbent material available from Nippon Shokubai under the trade name Aqualic L520 distributed onto the substrate layer in a non uniform layer having overall an average basis weight of 120 g/m², and a layer of thermoplastic material constituted by a hot melt adhesive available from HB Fuller under the trade name NV 1151 Zeropack applied in fibres having an average thickness of about 50 µm at a basis weight of 11 g/m². The absorbent core further comprises a substrate layer, constituted by a 65 g/m² latex bonded airlaid (LBAL) material comprising 30% by weight cellulose fibres, 40% by weight PET fibres and 30% by weight latex binder, available from Concert GmbH under the code WHXX65.

Chitosan lactate is uniformly distributed onto the garment facing surface of the substrate layer in a basis weight of 5 g/m²; the chitosan lactate can be for example applied as a 3% by weight aqueous solution uniformly sprayed onto the garment facing surface of the substrate layer in a suitable amount in order to have the desired basis weight of dry cationic polysaccharide upon evaporation of water.

The Rheological Creep Test and the Dynamical Mechanical Analysis (DMA) - Temperature Sweep Test mentioned hereinabove for measuring the cohesive strength parameter γ and the cross-over temperature parameter Tx respectively, are as described in the copending patent application EP 1447067, assigned to the Procter & Gamble Company.

### Artificial Menstrual Fluid (AMF)

Artificial Menstrual Fluid is based on modified sheep's blood that has been modified to ensure it closely resembles human menstrual fluid in viscosity, electrical conductivity, surface tension and appearance. It is prepared as explained in US Patent 6,417,424, assigned to The Procter & Gamble Company, from line 33 of column 17 to line 45 of column 18, to which reference is made.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent core (28) for an absorbent article (20) intended for absorption of menses or blood or vaginal discharges, said core (28) comprising a substrate layer (100),
said substrate layer (100) comprising a first surface and a second surface,
said absorbent core (28) further comprising a non uniform layer of absorbent polymer material (110),
said non uniform layer of absorbent polymer material (110) comprising a first surface and a second surface,
said absorbent core (28) further comprising a layer of a thermoplastic material (120),
said layer of thermoplastic material (120) comprising a first surface and a second surface,
wherein said second surface of said non uniform layer of absorbent polymer material (110) is in at least partial contact with said first surface of said substrate layer (100),
and wherein portions of said second surface of said, layer of thermoplastic material (110) are in direct contact with said first surface of said substrate layer (100) and portions of said second surface of said layer of thermoplastic material (120) are in direct contact with said first surface of said non uniform layer of absorbent polymer material (110),
said substrate layer (100) comprising a fibrous web of fibres,
**characterized in that**
said absorbent core (28) comprises a cationic polysaccharide (150), wherein said cationic polysaccharide (150) is comprised in a carrier material,
and wherein said layer of thermoplastic material (120) is a layer of fiberized hot melt adhesive,
said core (28) comprising front and rear transverse ends (160) and two longitudinal side ends (170), wherein said cationic polysaccharide material (150) is comprised along said longitudinal side ends (170).

2. An absorbent core (28) according to claim 1, further comprising a cover layer (130) comprising a first surface and a second surface, wherein said second surface of said cover layer (130) is in direct contact with said first surface of said layer of thermoplastic material (110).

3. An absorbent core (28) according to any preceding claim, wherein said cationic polysaccharide (150) is comprised within said non uniform layer of absorbent polymer material (110).

4. An absorbent core (28) according to claim 1, wherein said cationic polysaccharide (150) is comprised within said substrate layer (100).

5. An absorbent core (28) according to claim 1, wherein said non uniform layer of absorbent polymer material (110) does not extend up to said longitudinal side ends (170).

6. An absorbent core (28) according to any preceding claim, wherein said cationic polysaccharide material (150) is in particulate form.

7. An absorbent core (28) according to claim 17, wherein said carrier material comprises a mixture of polyethylene glycols.

8. An absorbent core (28) according to claim 17, wherein said carrier material is a polyethylenglycol having a molecular weight of at least 1200.

9. An absorbent core (28) according to any preceding claim, wherein said cationic polysaccharide (150) is comprised in a concentration of from 0.5 g/m² to 500 g/m², by weight of the cationic polysaccharide per square meter of the zone of application.

10. An absorbent article (20) comprising a liquid permeable topsheet (30), a backsheet (40), and an absorbent core (28) according to any preceding claim comprised therebetween.

## Patentansprüche

1. Absorptionskern (28) für einen Absorptionsartikel (20), der zur Absorption von Menstruationsflüssigkeit oder Blut oder Vaginalausfluss vorgesehen ist,
wobei der Kern (28) eine Substratschicht (100) umfasst,
wobei die Substratschicht (100) eine erste Oberfläche und eine zweite Oberfläche umfasst,
wobei der Absorptionskern (28) ferner eine nicht gleichförmige Schicht aus absorbierendem Polymermaterial (110) umfasst,
wobei die nicht gleichförmige Schicht aus absorbierendem Polymermaterial (110) eine erste Oberfläche und eine zweite Oberfläche umfasst,
wobei der Absorptionskern (28) ferner eine Schicht aus einem thermoplastischen Material (120) umfasst,
wobei die Schicht aus thermoplastischem Material (120) eine erste Oberfläche und eine zweite Oberfläche umfasst,
wobei die zweite Oberfläche der nicht gleichförmigen Schicht aus absorbierendem Polymermaterial (110) mindestens teilweise mit der ersten Oberfläche der Substratschicht (100) in Kontakt steht,
und wobei Abschnitte der zweiten Oberfläche der Schicht aus thermoplastischem Material (110) in direktem Kontakt mit der ersten Oberfläche der Substratschicht (100) stehen und Abschnitte der zweiten Oberfläche der Schicht aus thermoplastischem Material (120) in direktem Kontakt mit der ersten Schicht der nicht gleichförmigen Schicht aus absorbierendem Polymermaterial (110) stehen,
wobei die Substratschicht (100) eine Faserbahn von Fasern umfasst,
**dadurch gekennzeichnet, dass**
der Absorptionskern (28) ein kationisches Polysaccharid (150) umfasst, wobei das kationische Polysaccharid (150) in einem Trägermaterial enthalten ist,
und wobei die Schicht aus thermoplastischem Material (120) eine Schicht aus zerfasertem Heißschmelzkleber ist,
der Kern (28) vordere und hintere quer verlaufende Enden (160) und zwei längs verlaufende Seitenenden (170) umfasst, wobei das kationische Polysaccharidmaterial (150) entlang der längs verlaufenden Seitenenden (170) enthalten ist.

2. Absorptionskern (28) nach Anspruch 1, ferner umfassend eine Deckschicht (130), die eine erste Oberfläche und eine zweite Oberfläche umfasst, wobei die zweite Oberfläche der Deckschicht (130) in direktem Kontakt mit der ersten Oberfläche der Schicht aus thermoplastischem Material (110) steht.

3. Absorptionskern (28) nach einem der vorstehenden Ansprüche, wobei das kationische Polysaccharid (150) in der nicht gleichförmigen Schicht aus absorbierendem Polymermaterial (110) enthalten ist.

4. Absorptionskern (28) nach Anspruch 1, wobei das kationische Polysaccharid (150) in der Substratschicht (100) enthalten ist.

5. Absorptionskern (28) nach Anspruch 1, wobei sich die nicht gleichförmige Schicht aus absorbierendem Polymermaterial (110) nicht nach oben bis zu den längs verlaufenden Seitenenden (170) erstreckt.

6. Absorptionskern (28) nach einem der vorstehenden Ansprüche, wobei das kationische Polysaccharidmaterial (150) in Teilchenform vorliegt.

7. Absorptionskern (28) nach Anspruch 1, wobei das Trägermaterial eine Mischung von Polyethylenglycolen ist.

8. Absorptionskern (28) nach Anspruch 1, wobei das Trägermaterial ein Polyethylenglycol mit einem Molekulargewicht von mindestens 1200 ist.

9. Absorptionsartikel (28) nach einem der vorstehenden Ansprüche, wobei das kationische Polysaccharid (150) in einer Konzentration von 0,5 g/m² bis 500 g/m² bezogen auf das Gewicht des kationischen Polysaccharids pro Quadratmeter des Anwendungsbereichs enthalten ist.

10. Absorptionsartikel (20), umfassend eine flüssigkeitsdurchlässige Oberschicht (30), eine Unterschicht (40) und einen Absorptionskern (28) nach einem der vorstehenden Ansprüche, der dazwischen enthalten ist.

## Revendications

1. Noyau absorbant (28) pour un objet absorbant (20) destiné à l'absorption des règles ou de sang ou d'écoulements vaginaux, ledit noyau (28) comprenant une couche (100) de substrat,
ladite couche (100) de substrat comprenant une première surface et une deuxième surface,
ledit noyau absorbant (28) comprenant en outre une couche non uniforme de matériau polymère absorbant (110),
ladite couche non uniforme de matériau polymère absorbant (110) comprenant une première surface et une deuxième surface,
ledit noyau absorbant (28) comprenant en outre une couche de matériau thermoplastique (120),
ladite couche de matériau thermoplastique (120) comprenant une première surface et une deuxième surface,
ladite deuxième surface de ladite couche non uniforme de matériau polymère absorbant (110) étant en contact au moins partiel avec ladite première surface de ladite couche (100) de substrat,
et des parties de ladite deuxième surface de ladite couche de matériau thermoplastique (110) étant en contact direct avec ladite première surface de ladite couche (100) de substrat et des parties de ladite deuxième surface de ladite couche de matériau thermoplastique (120) étant en contact direct avec ladite première surface de ladite couche non uniforme de matériau polymère absorbant (110),
ladite couche (100) de substrat comprenant une toile fibreuse de fibres,
**caractérisé en ce que**
ledit noyau absorbant (28) comprend un polysaccharide cationique (150), ledit polysaccharide cationique (150) se trouvant dans un matériau support,
et ladite couche de matériau thermoplastique (120) étant une couche d'adhésif thermofusible défibré,
ledit noyau (28) présente des extrémités transversales, frontale et arrière (160) et deux extrémités longitudinales latérales (170), ledit matériau polysaccharide cationique (150) se trouvant le long desdites extrémités longitudinales latérales (170).

2. Noyau absorbant (28) selon la revendication 1, comprenant en outre une couche de recouvrement (130) comprenant une première surface et une deuxième surface, ladite deuxième surface de ladite couche de recouvrement (130) étant en contact direct avec ladite première surface de ladite couche de matériau thermoplastique (110).

3. Noyau absorbant (28) selon l'une quelconque revendication précédente, ledit polysaccharide cationique (150) se trouvant dans ladite couche non uniforme de matériau polymère absorbant (110).

4. Noyau absorbant (28) selon la revendication 1, ledit polysaccharide cationique (150) se trouvant dans ladite couche (100) de substrat.

5. Noyau absorbant (28) selon la revendication 1, ladite couche non uniforme de matériau polymère absorbant (110) ne s'étendant pas jusqu'auxdites extrémités longitudinales latérales (170).

6. Noyau absorbant (28) selon l'une quelconque revendication précédente, ledit matériau polysaccharide cationique (150) se trouvant sous forme particulaire.

7. Noyau absorbant (28) selon la revendication 1, ledit matériau support comprenant un mélange de polyéthylèneglycols.

8. Noyau absorbant (28) selon la revendication 1, ledit matériau support étant un polyéthylèneglycol présentant un poids moléculaire d'au moins 1200.

9. Noyau absorbant (28) selon l'une quelconque revendication précédentes, ledit polysaccharide cationique (150) se trouvant en une concentration de 0,5 g/m² à 500 g/m² en poids de polysaccharide cationique par mètre carré de la zone d'application.

10. Objet absorbant (20) comprenant une feuille supérieure perméable aux liquides (30), une feuille arrière (40) et un noyau absorbant (28) selon l'une quelconque revendication précédente se trouvant entre celles-ci.
